# EUROPEAN PATENT APPLICATION

(11) **EP 3 949 775 A1**
(43) Date of publication of application: **09.02.2022**
(21) Application number: 20782743.7
(22) Date of filing: 01.04.2020
(51) Int. Cl.: A41D 1/00, A41D 13/00

(54) **SPORTSWEAR CAPABLE OF PROVIDING ELECTRICAL STIMULATION**

(30) Priority: 02.04.2019 KR 20190038230; 30.03.2020 KR 20200038306
(71) Applicant: M20 Co., Ltd., Gangseo-gu Seoul 07528 (KR)
(72) Inventor: KIM, Jin Kil, Seoul 06099 (KR)
(74) Representative: Boden, Keith McMurray
(86) International application number: PCT/KR2020/004443
(87) International publication number: WO 2020/204591

(57) **Abstract**

Sportswear according to the present embodiment comprises: a plurality of conductive pads making contact with the user's skin so as to provide electrical stimulation to a plurality of locations on a user; a plurality of electrodes electrically connected to the respective pads; stretchable wires for transmitting electrical signals to the plurality of electrodes; a connector for connecting electrical signals to the stretchable wires; and a belt connected to the connector so as to provide externally provided electrical signals through the connector.

## Description

### [Technical Field]

The present invention relates to sportswear capable of providing electrical stimulation.

### [Background Art]

Sportswear according to the related art focuses on protecting a body of a user or not reducing an exercise function of the user by quickly discharging sweat discharged from the body. Thus, the sportswear focuses on functions of preventing sweat of the user from permeating the sportswear during exercise, draining moisture inside the sportswear to the outside and preventing external moisture from penetrating into the sportswear, preventing degradation of an exercise function with high elasticity, and the like.

Meanwhile, modern people who work in office work spend most of their time in offices. Most modern people are aware of the necessity of exercise. However, for reasons such as a lack of time, it is rare to perform an exercise repeatedly for as long as a recommended exercise time per day.

### [Disclosure]

### [Technical Problem]

Sportswear according to the related art only performs a passive function for preventing exercise ability of a user from being degraded and does not perform the role of performing an active function for improving the exercise ability of the user.

The present invention is directed to providing sportswear that may improve the exercise ability of the user beyond the functions of the sportswear according to the related art.

### [Technical Solution]

One aspect of the present invention provides sportswear according to the present embodiment including a plurality of conductive pads that are in contact with skin of a user to provide electrical stimulation to a plurality of locations on the user, a plurality of electrodes, each of which is electrically connected to one of the pads, stretchable conductive wires configured to transmit electrical signals to the plurality of electrodes, a connector configured to connect the electrical signals to the stretchable conductive wires, and a belt connected to the connector to provide the electrical signal provided from an outside through the connector.

The sportswear may have a form in which a top and bottoms are separated or the top and the bottoms are integrated and have a form of tights so as to be in close contact with the skin of the user.

The stretchable conductive wires may each include an electrically conductive line coated with an insulating material and disposed in a zigzag pattern, and an elastic member woven together with the electrically conductive line to provide stretchability.

Each of the electrodes may be any one of a rivet and an eyelet passing through the sportswear and the pads arranged on the sportswear and may further include a contact prevention film located on a surface on which each of the plurality of electrodes and the skin of the user are in contact with each other.

The belt may include a belt connector, the belt connector may be electrically connected to the connector, and the connector and the belt connector may be coupled by magnets having polarities that are complementary to each other.

The connector may include a front connector and a rear connector, and the belt connector may include a front belt connector coupled to the front connector and a rear belt connector coupled to the rear connector, wherein the front connector and the front belt connector may be coupled by magnets having corresponding polarities, and the rear connector and the rear belt connector may be coupled by magnets having polarities opposing to the magnets included in the front connector and the front belt connector.

The belt may further include a control panel connected to a signal source configured to provide the electrical signal so as to adjust an intensity of the electrical signal provided to the sportswear.

The conductive pads may be arranged at a plurality of locations selected from among a chest, an abdomen, flanks, thighs, arms, shoulders, flanks, and buttocks of the user.

### [Advantageous Effects]

According to the present embodiment, an electric signal is provided to a body of a user through a plurality of pads. Thus, muscles of the user can be contracted or relaxed with high efficiency.

### [Description of Drawings]

FIGS. 1A and 1B are views illustrating outlines of a front surface and a rear surface of sportswear according to the present embodiment.
FIG. 2 is a cross-sectional view schematically illustrating a cross section of the sportswear in which a pad is formed.
FIG. 3 is a view illustrating an outline of a stretchable wire included in the sportswear according to the present embodiment.
FIG. 4 is a view illustrating an outline of a belt.

### [Modes of the Invention]

Since the description of the present invention is merely an embodiment for structural or functional description, the scope of the present invention should not be interpreted as being limited by embodiments described in the text. That is, since the embodiments can be variously changed and have various forms, the scope of the present invention should be understood to include equivalents that can realize the technical spirit.

Meanwhile, the meaning of terms described in the present application should be understood as follows.

Terms such as "first" and "second" are intended to distinguish a first component from a second component, and the scope of rights should not be limited by these terms. For example, the first component may be referred to as the second component, and similarly, the second component may be referred to as the first component.

It should be understood that singular expressions include plural expressions unless the context clearly indicates otherwise, and it should be understood that terms such as "include" or "have" are intended to indicate that there are features, numbers, steps, operations, components, parts, or combinations thereof that are described in the specification and do not exclude in advance the possibility of the presence or addition of one or more other features, numbers, steps, operations, components, parts, or combinations thereof.

Terms "and/or" used to describe embodiments of the present invention is used to refer to each part and all parts. As an example, it should be understood that the expression "A and/or B" refers to "A, B, and both A and B."

In description of embodiments of the present invention, when it is determined that it is necessary to distinguish a plurality of elements that perform the same or similar functions, the description is made while adding symbols such as a, b, and c or 1, 2, and 3. However, when it is not necessary to distinguish the plurality of elements or the description is made while referring to all the elements, the description may be made while the added symbols are removed.

All terms used herein have the same meanings as those commonly understood by those skilled in the art to which the present invention belongs, unless otherwise defined. Terms defined in commonly used dictionaries should be interpreted as having the same meanings in the context of the related art and may not be interpreted as having ideal or excessively formal meanings, unless explicitly defined in the present application.

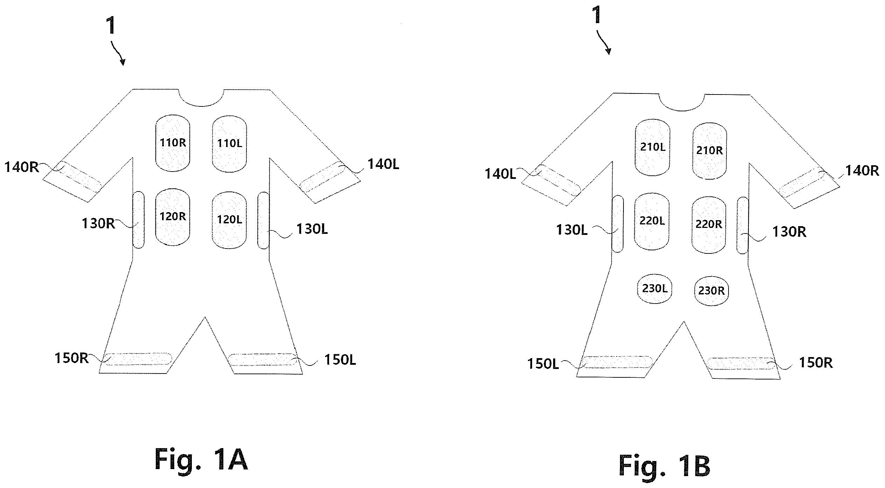

Hereinafter, sportswear according to the present embodiment will be described with reference to the accompanying drawings. FIGS. 1A and 1B are views illustrating outlines of a front surface and a rear surface of sportswear according to the present embodiment. A plurality of pads that may provide electrical signals to a user may be formed on sportswear 1 according to the present embodiment. Referring to FIG. 1A, pads 110L and 110R that may provide electrical signals to a left and right chest of the user and pads 120L and 120R that may provide electrical signals to a left and right abdomen of the user are located on the front surface of the sportswear 1. Pads 130L and 130R are located to provide electrical signals to the left and right flanks of the sportswear 1. Pads 140L and 140R are located to provide electric signals while surrounding left and right arms of the sportswear 1, and pads 150L and 150R are located to provide electrical signals while surrounding left and right thighs of the sportswear 1.

Referring to FIG. 1B, pads 210L and 210R that may provide electrical signals to left and right shoulder areas of the user and pads 220L and 220R that may provide electrical signals to left and right lower shoulder areas of the user are located on the rear surface of the sportswear 1. The pads 130L and 130R formed on the front surface extend from the left and right flanks of the rear surface of the sportswear 1. The pads 140L and 140R are located to surround the left and right arms of the sportswear 1, and the pads 150L and 150R are located to provide electrical signals while surrounding the left and right thighs of the sportswear 1. Pads 230L and 230R that provide electrical signals are located on left and right hips. Each of the pads is formed of a conductive material and provides an electrical signal provided by a signal source (not illustrated) through skin of the user.

In the embodiment illustrated in FIGS. 1A and 1B, it is illustrated that the sportswear 1 is a pair of tights in which a top and bottoms are attached and is wearable through a zipper (not illustrated) formed on a front surface thereof. In an embodiment not illustrated, the sportswear may have a form in which a top and bottoms are separated.

The sportswear 1 has a form of tights that are in close contact with the skin of the user, thereby improving contact between the pads and the skin of the user. Further, the sportswear is formed of a material that may not absorb but rather discharges sweat discharged by the user during exercise like sportswear according to the related art and thus does not reduce the mobility of the user.

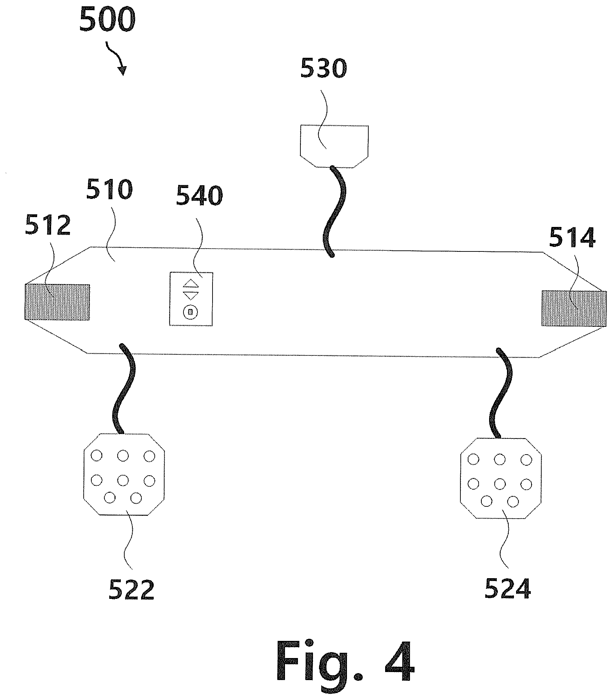

The sportswear 1 may include a connector (not illustrated) electrically connected to the pads located on the front surface and the rear surface thereof through a stretchable wire. As an example, the connector may include a front connector connected to the pads arranged on the front surface of the sportswear 1 and a rear connector connected to the pads arranged on the rear surface of the sportswear 1. As another example, the sportswear 1 may have a single connector and may be electrically connected to the pads arranged on the front surface and the pads arranged on the rear surface through the connector. In one embodiment, the connector may include a magnet and may be connected to a connector 520 (see FIG. 4) formed on a belt 500 and having an opposing polarity. An electrical signal is transmitted through the connector located in the sportswear 1, and an electrical signal is provided to the user through the pads connected to the connector through the stretchable conductive wire 400.

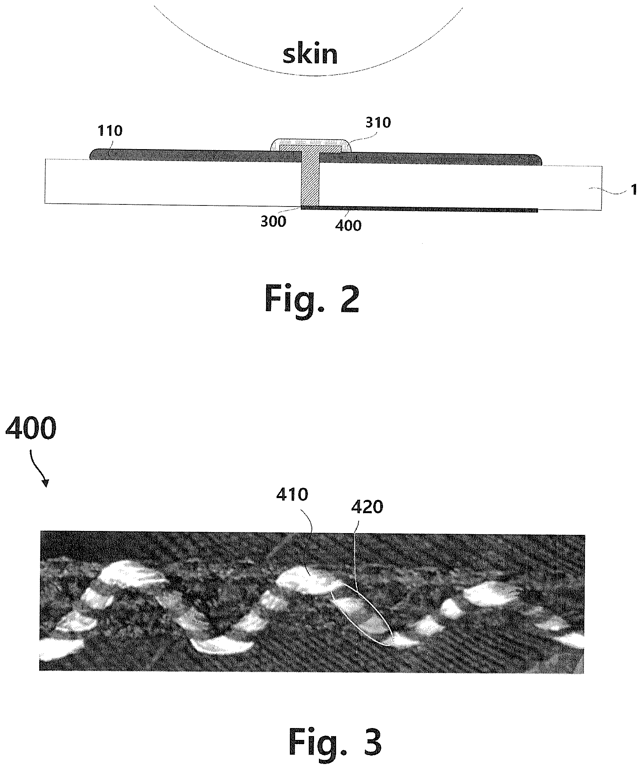

FIG. 2 is a cross-sectional view schematically illustrating a cross section of the sportswear 1 in which a pad 110 is formed. Referring to FIG. 2, the pad 110 is formed of a conductive material and is disposed on the sportswear 1 through sewing and/or bonding. As described above, the pad 110 is in contact with the skin of the user to transmit, to the user, the electrical signal transmitted from the stretchable conductive wire 400.

The electrode 300 is formed of a conductive metal material and transmits, to the pad 110, the electrical signal transmitted from the stretchable conductive wire 400 disposed on the other surface of the sportswear 1. In one embodiment, the electrode may have the form of a rivet, an eyelet, and the like that passes through the sportswear 1 to connect the pad 110 and the stretchable conductive wire 400.

In one embodiment, since the electrode 300 is formed of a conductive metal material, metal allergies may occur when the electrode 300 comes into contact with the skin of the user. Thus, a contact prevention film 310 may be formed in a portion in which the electrode 300 is in contact with the skin of the user so that the skin and the electrode 300 are not in contact with each other. The contact prevention film 310 may be disposed on the pad 110 through sewing or may be disposed to adhere to the electrode 300 and the pad 110.

FIG. 3 is a view illustrating an outline of the stretchable conductive wire 400 included in the sportswear 1 according to the present embodiment. Referring to FIG. 3, the stretchable conductive wire 400 includes an insulation-coated conductive wire 410 disposed in a zig-zag pattern and an elastic member 420 in which a conductive wire is woven together with the insulation-coated conductive wire to provide stretchability.

The insulation-coated conductive wire 400 may be a stranded wire and may be insulated by being individually coated with an insulating material. As an example, the insulation-coated conductive wire 400 may be a nichrome wire coated with an insulating material. As another example, the insulation-coated conductive wire 400 may be a single wire.

The elastic member 420 is a member having elasticity so that the insulation-coated conductive wire 410 disposed in a zigzag pattern is stretched and may then return to an original position. As an example, the elastic member 420 may be a rubber band coated with an outer skin such as cotton. The stretchable conductive wire 400 may be sewn while forming the sportswear 1 and being disposed in the sportswear 1.

When the user moves an arm or a leg while wearing the sportswear 1 according to the present embodiment and exercising, the sportswear 1 may be stretched or contracted. In this process, despite the movement of the user, the stretchable conductive wire 400 may be stretched and return to an original state again. Thus, the sportswear 1 according to the present embodiment may provide electrical stimulation that may enhance the exercise effect without interfering with the exercise of the user.

FIG. 4 is a view illustrating an outline of the belt 500. Referring to FIG. 4, the belt 500 includes a belt body 510, fixing members 512 and 514 that fix both ends of the belt, connectors 522 and 524 coupled to the connector formed in the sportswear, a signal connector 530 connected to a signal source (not illustrated), and a control panel 540 that controls an electrical signal provided to a human body.

The fixing members 512 and 54 fix the belt 500 to be fitted onto a human body of the user. In the illustrated embodiment, the fixing members 512 and 514 may be velcro, and the user may fix the velcro to be fitted onto his/her body after wearing the belt. As another example, the fixing member 514 may be a hole drilled at regular intervals, and the fixing member 514 may be a pin inserted into the hole to fix the belt. After wearing the belt, the user may fix the belt to be fitted onto his/her body using the pin and the hole.

The connectors 522 and 524 are coupled to the connector located in the sportswear 1. In one embodiment, the connector formed in the sportswear and the connectors 522 and 524 disposed on the belt 500 may have magnetism, and an electrical connection may be made through the connector only with the matching magnetisms. For example, the connector connected to the front pads of the sportswear 1 may have an N-pole magnetism, the connector formed in the belt to provide an electrical signal to the front pads of the sportswear may have an S-pole magnetism, and thus coupling may be made. Further, the connector connected to the rear pads of the sportswear 1 may have an S-pole magnetism, the connector formed in the belt to provide an electrical signal to the rear pads of the sportswear may have an N-pole magnetism, and thus the coupling may be made. Thus, a misconnection of connectors can be prevented.

The control panel 540 controls the electrical signal provided to the body of the user. In one embodiment, the user may increase the magnitude of the electrical signal by pressing a positive triangle button located on the control panel 540 and may decrease the magnitude of the electrical signal by pressing a negative triangle button. Further, the user may stop providing the electrical signal to the user by pressing a stop button located on the control panel 540.

The signal connector 530 may be connected to an external signal source (not illustrated) to receive the electrical signal to be provided to the user and may provide, to the external signal source, a control signal corresponding to the control of the control panel 540 by the user.

As described above, according to the sportswear 1 according to the present embodiment, the electrical signal is provided to the skin of the user, and thus there is provided an effect of enhancing an exercise effect without disturbing the mobility of the user during exercise.

Although the embodiments illustrated in the drawings have been described to help understanding of the present invention, these are embodiments for implementation and are merely illustrative, and those skilled in the art may thus understand that various modifications and equivalent other embodiments may be derived from the description. Thus, the true technical scope of the present invention should be determined by the appended claims.

## Claims

1. Sportswear comprising:
a plurality of conductive pads that are in contact with skin of a user to provide electrical stimulation to a plurality of locations on the user;
a plurality of electrodes electrically, each of which is connected to one of the pads;
stretchable conductive wires configured to transmit electrical signals to the plurality of electrodes;
a connector configured to connect the electrical signals to the stretchable conductive wires; and
a belt connected to the connector to provide the electrical signal provided from an outside through the connector.

2. The sportswear of claim 1, wherein the sportswear has a form in which a top and bottoms are separated or the top and the bottoms are integrated and has a form of tights to be in close contact with the skin of the user.

3. The sportswear of claim 1, wherein the stretchable conductive wires include:
an electrically conductive line coated with an insulating material and disposed in a zigzag pattern; and
an elastic member woven together with the electrically conductive line to provide stretchability.

4. The sportswear of claim 1, wherein each of the electrodes is any one of a rivet and an eyelet passing through the sportswear and the pads arranged on the sportswear and further includes a contact prevention film located on a surface on which each of the plurality of electrodes and the skin of the user are in contact with each other.

5. The sportswear of claim 1, wherein the belt includes a belt connector,
wherein the belt connector is electrically connected to the connector, and the connector and the belt connector are coupled by magnets having polarities that are complementary to each other.

6. The sportswear of claim 5, wherein the connector includes a front connector and a rear connector, and
the belt connector includes a front belt connector coupled to the front connector and a rear belt connector coupled to the rear connector,
wherein the front connector and the front belt connector are coupled by magnets having corresponding polarities, and
the rear connector and the rear belt connector are coupled by magnets having polarities opposing to the magnets included in the front connector and the front belt connector.

7. The sportswear of claim 1, wherein the belt further includes a control panel connected to a signal source configured to provide the electrical signal so as to adjust an intensity of the electrical signal provided to the sportswear.

8. The sportswear of claim 1, wherein the conductive pads are arranged at a plurality of locations selected from among a chest, an abdomen, flanks, thighs, arms, shoulders, flanks, and buttocks of the user.
